(19) Europäisches Patentamt
European Patent Office
Office européen des brevets

(11) **EP 3 595 449 B1**

(12) **EUROPÄISCHE PATENTSCHRIFT**

(45) Veröffentlichungstag und Bekanntmachung des Hinweises auf die Patenterteilung:
**24.01.2024 Patentblatt 2024/04**

(21) Anmeldenummer: **18718088.0**

(22) Anmeldetag: **07.03.2018**

(51) Internationale Patentklassifikation (IPC):
**A01N 43/36** *(2006.01)*    **A01N 37/24** *(2006.01)*
**A01N 43/56** *(2006.01)*    **A01N 43/54** *(2006.01)*
**A01N 63/30** *(2020.01)*    **A01P 3/00** *(2006.01)*

(52) Gemeinsame Patentklassifikation (CPC):
(C-Sets verfügbar)
**A01N 43/36; A01N 63/30**     (Forts.)

(86) Internationale Anmeldenummer:
**PCT/AT2018/000012**

(87) Internationale Veröffentlichungsnummer:
**WO 2018/165686 (20.09.2018 Gazette 2018/38)**

(54) **ZUBEREITUNG ENTHALTEND WENIGSTENS FLUDIOXONIL UND EINE MISCHUNG ENTHALTEND AUREOBASIDIUM PULLULANS STÄMME**

PREPARATION COMPRISING AT LEAST FLUDIOXONIL AND A MIXTURE COMPRISING AUREOBASIDIUM PULLULANS STRAINS

PRÉPARATION COMPRENANT AU MOINS FLUDIOXONIL ET UN MÉLANGE COMPRENANT DES SOUCHES D'AUREOBASIDIUM PULLULANS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.03.2017 AT 1042017**

(43) Veröffentlichungstag der Anmeldung:
**22.01.2020 Patentblatt 2020/04**

(73) Patentinhaber: **SAN Agrow Holding GmbH**
**3130 Herzogenburg (AT)**

(72) Erfinder:
• **BINDER, Eva-Maria**
**3430 Tulln (AT)**
• **DONAT, Christina Maria**
**3465 Königsbrunn am Wagram (AT)**

(74) Vertreter: **Cunow, Gerda**
**Cunow Patentanwalts KG**
**Engerthstraße 146/3/1**
**1200 Wien (AT)**

(56) Entgegenhaltungen:
EP-A2- 0 630 569     WO-A1-2014/016279
WO-A1-2016/001125     WO-A2-2008/055614
WO-A2-2008/114304

(52) Gemeinsame Patentklassifikation (CPC): (Forts.)

C-Sets
**A01N 43/36, A01N 43/54;**
**A01N 63/30, A01N 37/24, A01N 43/36,**
**A01N 43/54, A01N 43/56;**
**A01N 63/30, A01N 43/36, A01N 43/54;**
**A01N 63/30, A01N 63/30, A01N 37/24,**
**A01N 43/36, A01N 43/54, A01N 43/56;**
**A01N 63/30, A01N 63/30, A01N 43/36, A01N 43/54**

**Beschreibung**

Die vorliegende Erfindung bezieht sich auf eine Zubereitung enthaltend wenigstens ein chemisches Fungizid sowie die Verwendung der Zubereitung und Verfahren zur Prophylaxe oder zur Reduktion der Ausbreitung von wenigstens einer durch einen pilzlichen Pathogen ausgelösten Pflanzenerkrankung.

Um Ernteausbeuten in der Landwirtschaft so weit wie möglich maximieren zu können, ist es erforderlich, landwirtschaftliche Produkte vor Schädlingsbefall so gut wie möglich zu schützen. Dies ist in allen landwirtschaftlichen Kulturen, insbesondere im Obst- und Gemüseanbau von großer Bedeutung und es ist zum Schutz der Kulturpflanzen vor Befall durch Pathogene üblich, diese mit chemischen Fungiziden zu spritzen bzw. zu behandeln, um eine pflanzenpathogene Infektion zu verhindern bzw. deren Ausbreitung zu reduzieren. Derartige chemische Fungizide haben üblicherweise ein sehr breites Anwendungsspektrum und wirken nicht spezifisch gegen einzelne Pathogene, so dass sie sehr weit verbreitet eingesetzt werden. Ähnlich wie dies von Antibiotika bekannt ist, entwickeln die Pathogene gegen derartige fungizid wirkende chemische Substanzen im Laufe der Zeit Resistenzen bzw. eine verringerte Sensitivität und die Wirkung der für die Behandlung bzw. Vorbeugung gegen Befall mit Pathogenen eingesetzten chemischen Fungizide wird immer geringer, bis die pathogenen Stoffe den Spritzmitteln gegenüber soweit resistent sind, dass eine neue Substanz gesucht werden muss.

Die chemischen Fungizide weisen neben dem Problem, dass im Laufe der Zeit Resistenzen bzw. verringerte Sensitivitäten gegen sie entwickelt werden, auch das Problem auf, dass diese nach Ausbringen auf die zu behandelnden Pflanzen wenigstens teilweise in den Boden gelangen, wodurch es zu Kontaminierungen kommen kann und neben den Kontaminierungen auch weitere Mikroorganismen, deren Behandlung bzw. Vorbeugung im vorliegenden Fall gar nicht beabsichtigt war, ebenfalls gegenüber diesen chemischen Fungiziden resistent werden können. Auf diese Weise tritt der unerwünschte Effekt ein, dass nicht nur die Zielorganismen (Pathogene) resistent werden können, sondern auch Nicht-Zielorganismen, deren Behandlung durch den Einsatz des jeweiligen chemischen Fungizids ursprünglich nicht beabsichtigt war. Dies kann zur Folge haben, dass diese Nicht-Zielorganismen ihrerseits völlig unerkannt Resistenzen bzw. verringerte Sensitivitäten ausbilden, und dann im erforderlichen Fall nicht mit herkömmlichen chemischen Fungiziden behandelt werden können bzw. eine Behandlung nicht den erwünschten Erfolg mit sich bringen wird.

Derartig behandelte Pflanzen, Obst oder Gemüse gelangen in der Folge in den Nahrungskreislauf von Nutztieren und Menschen und können aufgrund der nicht bekannten Belastung mit Fungiziden große Schäden hervorrufen, indem beispielsweise sekundäre Resistenzen ausgebildet werden.

Darüber hinaus steht dem Einsatz von chemischen Fungiziden die öffentliche Meinung immer negativer gegenüber, da die Menschen aufgrund ihres immer größer werdenden Wissens betreffend die Wirkungsweise und die Folgen übermäßiger Verwendung von chemischen Fungiziden, den Einsatz derselben ablehnen und soweit als möglich zurückdrängen wollen, was die Suche nach Alternativen erforderlich macht.

Neben dem Einsatz von chemischen Fungiziden ist es bereits seit einiger Zeit bekannt, biologische Fungizide zum Einsatz zu bringen, welche den Vorteil haben, dass die Pathogene keine Resistenzen gegen diese biologischen Fungizide ausbilden können. Ob und inwieweit die Wirkung von biologischen Agentien jener von chemischen Fungiziden gleichgesetzt werden kann, hängt von dem speziellen eingesetzten, biologischen Mittel bzw. der biologischen Mischung ab und von dem speziellen Pathogen, gegen welchen eine Wirkung erzielt werden soll, so dass für jeden Einzelfall eine Mehrzahl von Tests und Untersuchungen erforderlich ist.

Aus der DE 699 19 762 T2 sind fungizide Zusammensetzungen zur Bekämpfung von phytopathogenen Erkrankungen auf Nutzpflanzen bekannt geworden, bei welchen eine Kombination von zwei fungizid wirkenden Substanzen zum Einsatz gebracht wurde, um auf diese Weise eine synergistische fungizide Wirkung zu erreichen und dadurch die Gesamtmenge an eingesetzten fungiziden Stoffen absenken zu können.

Weiterhin ist der EP 0 930 824 B1 eine Mischung entnehmbar, welche im Wesentlichen aus einer Aufbereitung der Pflanze Reynoutria sachalinensis sowie einem Stickstoffdünger besteht, welche Mischung zur Verhütung des Befalls durch pflanzenpathogene Pilze und zur Bekämpfung von pflanzenpathogenen Pilzen einsetzbar ist. Auch bei dieser Mischung soll die Gesamtmenge an ausgebrachten Wirkstoffen verringert werden.

Der WO 99/62341 ist schließlich eine schützende und heilende Zusammensetzung zu entnehmen ebenso wie entsprechende Zusammensetzungen, welche Pflanzenerkrankungen biologisch behandeln sollen, wobei die Zusammensetzungen insbesondere für die Verwendung nach der Ernte gedacht sind. Eine derartige Zusammensetzung enthält hierbei wenigstens einen antagonistisch wirkenden Mikroorganismus und ein insbesondere biologisches Antipilzmittel, wie z.B. ein Enzym.

Schließlich haben R. Castoria et al. (Postharvest Biology and Technology 22, Seite 7 bis 17, 2001) die Wirkung von *Aureobasidium pullulans* (LS-30) als einen Antagonisten von pathogenen Stoffen auf Früchten, welche diese nach der Ernte befallen, beschrieben, in welcher festgestellt wurde, dass *Aureobasidium pullulans* eine signifikante antagonistische Wirksamkeit gegen *Botrytis cinerea, Penicillium expansum, Rhizopus stolonifer* und *Aspergillus niger* bei verschiedenen Pflanzen zeigt. So wurden unter anderem Apfelwunden mit *Aureobasidium pullulans* (LS-30) behandelt und zum Vergleich Wunden nur mit Wasser behandelt. Diese Ergebnisse zeigten, dass *Aureobasidium pullulans* eine gute antagonistische Wirksamkeit gegen pilzliche Apfelfäule-Erreger hat.

WO 2008/114304 beschreibt die Anwendung von *Aureobasidium pullulans* Stämmen gemeinsam mit dem Adjuvantien Kalziumpropionat und Kalziumpropionat mit Sojabohnenöl bzw. mit dem Fungizid Procymidone zur Bekämpfung von *Botrytis cinerea* bei Weintrauben. Es wurden allerdings weder eine Kombination aus einzelnen *Aureobasidium pullulans* Stämmen noch aus einem *Aureobasidium pullulans* Stamm mit dem Fungizid Fludioxonil bzw. einer Mischung aus Cyprodinil, Fludioxonil getestet.

Schließlich wurde auf der Homepage der Firma BIOFA [http://www.biofa-profi.de/de/b/blossomprotecttm.html?file=files/content/Prdukte/BlossomProtectTM/blosso mprotect produkthandbuch 2012.pd] bereits eine Kombination aus Cyprodinil und *Aureobasidium pullulans* DSM 14940 und DSM 14941 nahegelegt. Der in diesem Dokument ebenfalls enthaltenen Mischbarkeitsliste ist jedoch eine Vielzahl von chemischen Fungiziden entnehmbar, welche aufgrund ihrer Nicht-Mischbarkeit mit dem *Aureobasidium pullulans* DSM 14940 und DSM 14941 enthaltenden Produkt Blossom Protect™ bzw. Boni Protect™ in den in diesem Dokument beschriebenen Zubereitungen nicht zum Einsatz gelangen können.

Trotz dieser Erkenntnisse besteht weiterhin das Erfordernis, weitere wirksame Fungizide bzw. fungizide Mischungen zur Verfügung zu stellen, mit welchen die Einsatzmenge chemischer Fungizide deutlich herabgesetzt werden kann und mit welchen darüber hinaus eine deutliche verbesserte Wirkung gegenüber dem Einsatz einer Einzelsubstanz, und zwar sowohl wenigstens eines chemischen Fungizides als auch eines biologischen Wirkstoffs erreicht werden kann.

Zur Lösung dieser Aufgabe ist die Zubereitung gemäß der vorliegenden Erfindung im Wesentlichen dadurch gekennzeichnet, dass zusätzlich zu dem wenigstens einen chemischen Fungizid eine Mischung enthaltend wenigstens die Stämme *Aureobasidium pullulans* DSM 14940 und DSM 14941 enthalten ist, wobei das wenigstens eine chemische Fungizid sowie die Mischung der Stämme *Aureobasidium pullulans* Stämme in synergistisch wirkenden Mengen in der Zubereitung vorliegen und dass das wenigstens eine chemische Fungizid aus Fludioxonil oder einer Kombination enthaltend Fludioxonil und Cyprodinil gewählt ist. Indem die Zubereitung zusätzlich zu dem wenigstens einem chemischen Fungizid eine Mischung enthaltend wenigstens die Stämme *Aureobasidium pullulans* DSM 14940 und DSM 14941 in synergistisch wirksamen Mengen enthält, gelingt es, eine bessere fungizide Wirkung zu erzielen, als wenn die Einzelsubstanzen alleine bzw. gesondert eingesetzt worden wären. Schließlich gelingt es mit einer derartigen Vorgangsweise, Resistenzen gegen die chemischen Fungizide bestehend aus Fludioxonil oder einer Kombination enthaltend Fludioxonil und Cyprodinil, welche bei deren alleinigen Einsatz bereits beobachtet werden, aufzuheben bzw. hintanzuhalten und so die Wirkungsweise der Fungizide in überraschender Weise wieder deutlich zu verbessern. Besonders wirksam ist die Zusammensetzung bei der Prophylaxe von pilzlichen Pathogenen die zumindest eine Resistenz bzw. eine geringere Sensitivität gegenüber wenigstens einem der chemischen Fungizide in der Zubereitung aufweisen. Besonders wirksam bedeutet, dass der Synergismusfaktor der Zubereitung gegenüber den Einzelsubstanzen größer als 1,1 ist.

Insbesondere bevorzugt ist eine Kombination aus Fludioxonil und Cyprodinil Insbesondere Cyprodinil oder Fludioxonil zeigen gegenwärtig bereits eine deutlich eingeschränkte Wirksamkeit gegenüber pilzlichen Pathogenen, da diese bereits Resistenzen gegen diese zwei Wirkstoffe entwickeln. Überraschender Weise konnte gezeigt werden, dass, wenn eine Kombination der chemischen Fungizide Fludioxonil und Cyprodinil in einer Zubereitung gemäß der Erfindung enthalten ist, die Resistenzen unterdrückt werden können und die Substanzen, selbst wenn sie in geringen Konzentrationen in der Zubereitung enthalten sind, ihre volle Wirkung gegenüber pilzlichen Pathogenen entwickeln können.

Unter dem Begriff "Zubereitung" wird eine fertige Lösung oder Suspension verstanden, die alle Einzelsubstanzen, nämlich zumindest ein chemisches Fungizid und die beiden *Aureobasidium pullulans* Stämme DSM 14940 und DSM 14941 beinhaltet und die direkt auf Pflanzen aufgebracht wird. Typischerweise handelt es sich bei Zubereitungen um wässrige Lösungen oder Suspensionen, auch Tankmischungen genannt, die üblicherweise vor Aufbringung auf die Pflanzen abgemischt werden. Dabei werden die Einzelsubstanzen bzw. -komponenten in konzentrierter flüssiger oder fester Form mit Wasser in einem Tank versetzt und gelöst bzw. suspendiert. Dabei ist es unerheblich ob die Einzelsubstanzen bzw. -komponenten separat, oder als eine konzentrierte Vormischung bestehend aus zumindest einem chemischen Fungizid und den beiden *Aureobasidium pullulans* Stämmen DSM 14940 und DSM 14941, in den Tank eingebracht, mit Wasser versetzt und gelöst bzw. suspendiert werden. Die Reihenfolge in der Wasser und die Einzelsubstanzen bzw. -komponenten oder die Vormischung in den Tank eingebracht werden ist dabei unerheblich. Dabei werden die Einzelsubstanzen bzw. -komponenten oder die konzentrierten Vormischungen zum Beispiel um den Faktor 1:10, 1:100, 1:1000 oder 1:10000 im Tank verdünnt, um die wirksame Konzentration in der Zusammensetzung zu erreichen. Überraschenderweise ist es mit den vorliegenden Zubreitungen nunmehr gelungen, eine Mischung von Fludioxonil bzw. von Fludioxonil und Cyprodinil mit den den beiden *Aureobasidium pullulans* Stämmen DSM 14940 und DSM 14941 herzustellen, obwohl bekannte diese Stämme bzw. dies chemischen Fungizide enthaltende Produkte in der Literatur als nicht mischbar beschrieben wurden (siehe Mischbarkeitsliste als Anlage zu [http://www.biofa-profi.de/de/b/blossomprotecttm.html?file=files/content/Prdukte/BlossomProtectTM/blosso mprotect produkthandbuch 2012.pd]).

Unter dem Begriff "chemisches Fungizid" wird ein chemischer, fungizider Wirkstoff verstanden, insbesondere Cyprodinil (CAS-Nr. 121552-61-2), Fludioxonil (CAS-Nr. 131341-86-1), wobei zumindest Fludioxonil zwingend als wenigstens ein chemisches Fungizid in der Zubereitung enthalten ist.

Unter dem Begriff "pilzlicher Pathogen" wird hier ein Mikroorganismus verstanden, der in seinem Wirt eine Krankheit verursacht. Pilzliche Pathogene sind hierbei vorzugsweise die Mikroorganismen Neofabreaea spp., insbesondere *Pezicula malicorticis* (DSMZ 62715) und Botrytis spp., insbesondere *Botrytis cinerea,* welche Krankheiten bei Obst-, Wein- und Gemüsekulturen, insbesondere Äpfeln, Weintrauben, Erdbeeren, und Kürbisgewächsen, wie Zucchini und Speisekürbissen hervorrufen.

Eine besonders gute synergistische Wirkung wird dadurch erreicht, dass in der Zubereitung das wenigstens eine chemische Fungizid sowie eine Mischung enthaltend wenigstens die Stämme *Aureobasidium pullulans* DSM 14940 und DSM 14941 in synergistisch wirkenden Mengen enthält. Überraschender Weise hat sich herausgestellt, dass der Einsatz bzw. die Anwendung von einem chemischen Fungizid, enthaltend wenigstens Fludioxonil und der Mischung enthaltend wenigstens zwei voneinander verschiedene *Aureobasidium pullulans* Stämme, nämlich DSM 14940 und DSM 14941, eine deutlich bessere, insbesondere eine synergistische, Wirksamkeit zeigt, im Gegensatz zur Anwendung von nur einem der beiden *Aureobasidium pullulans* Stämme und dem chemischen Fungizid. Das Zellmengenverhältnis der zwei *Aureobasidium pullulans* Stämme DSM 14940 und DSM 14941 zueinander ist hierbei zwischen 2:1 und 1:2, insbesondere 1:1 gewählt, so dass eine entsprechende Mischung auch einfach herstellbar ist.

Eine besondere zuverlässige, insbesondere synergistische Wirkung der Zubereitung wird dadurch erzielt, wenn die *Aureobasidium pullulans* Stämme DSM 14940 und DSM 14941 jeweils in einer Konzentration von $1 \times 10^5$ bis $1 \times 10^8$ Zellen/ml Zubereitung vorliegen. Indem die *Aureobasidium pullulans* Stämme DSM 14940 und DSM 14941 jeweils in einer Konzentration von $1 \times 10^5$ bis $\times 10^8$ Zellen/ml Zubereitung, vorzugsweise $1 \times 10^6$ bis $2 \times 10^7$ Zellen/ml Zubereitung, vorzugsweise $1{,}67 \times 10^6$ bis $3{,}33 \times 10^6$ Zellen/ml Zubereitung, insbesondere bevorzugt von $2{,}5 \times 10^6$ Zellen/ml Zubereitung enthalten sind, gelingt es bei Anwendung dieser Zubereitung, Fäulnis, die durch verschiedene pilzliche Pathogene gewählt aus der Gruppe Neofabreaea spp., insbesondere *Pezicula malicorticis* (DSMZ 62715) und Botrytis spp., insbesondere *Botrytis cinerea,* Monilinia spp., Penicillium spp., Coletotrichum spp. u.a. sicher und zuverlässig zu unterdrücken, insbesondere die Vergrößerung von Fäulnisflecken, die durch äußere Verletzungen der Früchte bewirkt werden, hintanzuhalten.

Gemäß einer Weiterbildung der Erfindung ist die Zubereitung hierbei so ausgebildet, dass als synergistisch wirkende Mengen Cyprodinil in einer Konzentration von 0,00375 g/l Zubereitung bis 5 g/l Zubereitung, vorzugsweise 0,02 g/l Zubereitung bis 1,0 g/l Zubereitung, insbesondere 0,1875 g/l Zubereitung bis 0,375 g/l Zubereitung enthalten ist und dass Fludioxonil in einer Konzentration von 0,0025 g/l Zubereitung bis 5 g/l Zubereitung, vorzugsweise 0,01 g/l Zubereitung bis 1,0 g/l Zubereitung, insbesondere 0,125 g/l Zubereitung bis 0,25 g/l Zubereitung enthalten ist. Die eingesetzte Menge an chemischen Fungiziden hängt vor allem davon ab, ob der pilzliche Pathogen bereits eine Resistenz oder eine verminderte Empfindlichkeit bzw. Sensitivität gegenüber dem wenigstens einen chemischen Fungizid aufweist. Wenn die chemischen Fungizide Cyprodinil oder Fludioxonil in den angegebenen Mengen in der Zubereitung enthalten sind, können sie trotz der geringen enthaltenen Menge ihre Wirkung gegenüber pilzlichen Pathogenen voll entfalten und überraschender Weise werden von den pilzlichen Pathogenen gegenüber diesen bekannten chemischen Fungiziden in Kombination mit *Aureobasidium pullulans* Stämmen DSM 14940 und DSM 14941 keine bzw. verringerte Resistenzen oder Sensitivitäten gezeigt. Darüber hinaus zeigte die Zubereitung gemäß der Erfindung, welche die Mischung aus *Aureobasidium pullulans* Stämme DSM 14940 und DSM 14941 enthält, eine synergistische Wirkung, so dass mit einer Einsatzmenge, die deutlich geringer als die Summe der Einsatzmengen von den jeweiligen Einzelsubstanzen bzw. -komponenten, welche für das Erreichen einer ebenso guten Wirkung erforderlich gewesen wären, ist, das Auslangen gefunden wird.

Unter synergistischer Wirkung wird im vorliegenden Fall eine über-additive Steigerung der fungiziden Wirkung einer Kombination von wenigstens einem chemischen Fungizid mit einer Mischung der beiden *Aureobasidium pullulans* Stämmen DSM 14940 und DSM 14941 gegenüber a) der alleinigen Anwendung des wenigstens einem chemischen Fungizids bzw. gegenüber b) der alleinigen Anwendung der Mischung der beiden *A. pullulans* Stämme DSM 14940 und DSM 14941 bzw. gegenüber c) der Anwendung des wenigstens einem chemischen Fungizids gemeinsam mit leidglich einem der beiden *A. pullulans* Stämme DSM 14940 oder DSM 14941 verstanden. Der Einsatz der *A. pullulans* Stämme DSM 14940 und DSM 14941 einzeln in Kombination mit chemischen Fungiziden bzw. der Einsatz von Mischungen von herkömmlicher *A. pullulans* Stämme (AP 241 und AP 298) miteinander oder mit einem der beiden A. *pullulans* Stämme DSM 14940 und DSM 14941 in Kombination mit chemischen Fungiziden zeigte ebenfalls überraschenderweise keine synergistische Wirkung. Zur mathematischen Berechnung der synergistischen Wirkung wird wie in Colby et al. (Weeds 15, Seite 20 bis 22, 1967) vorgegangen, wobei ein Synergismusfaktor von größer 1 als Bestätigung des Vorliegens einer synergistischen Wirkung gesehen wird.

Die beste Wirkung konnte dadurch erzielt werden, dass eine Zubereitung gemäß der Erfindung im Wesentlichen dadurch gekennzeichnet ist, dass Cyprodinil und Fludioxonil in einem Gewichtsverhältnis von 2:1 bis 1:2, vorzugsweise von 2:1 bis 1:1, insbesondere von 1,5:1 in der Zubereitung vorliegen. Indem in der Zubereitung neben zwei biologischen Fungiziden (=*A. pullulans* Stämme DSM 14940 und DSM 14941) auch zwei chemische Fungizide eingesetzt sind, gelingt nicht nur eine Breitbandwirkung gegen pilzliche Pathogene, sondern überraschender Weise hat sich gezeigt, dass die Einsatzmenge der Einzelsubstanzen drastisch abgesenkt werden konnte, so dass eine synergistische Wirkung sämtlicher Bestandteile nachgewiesen ist.

Die Erfindung zielt weiterhin auf die Verwendung einer Zubereitung gemäß der vorliegenden Erfindung ab, welche zur Prophylaxe und/oder zur Eindämmung der Ausbreitung von durch pilzliche Pathogene verursachten Pflanzenerkrankungen eingesetzt wird.

Mit einer derartigen Verwendung gelingt es, die Entstehung und/oder Ausbreitung von Pflanzenerkrankungen auf den betroffenen Früchten einzudämmen bzw. vollständig zum Stillstand zu bringen, wodurch eine längere Lagerzeit der Ernte gewährleistet werden kann und insbesondere die Ausbreitung von pilzlichen Pathogenen unterdrückt werden kann.

Insbesondere bevorzugt wird die Zubereitung bei Pflanzenerkrankungen angewandt, die durch die pilzlichen Pathogene gewählt aus der Gruppe Neofabreaea spp, insbesondere *Neofabraea malicoricis* bzw. *Pezicula malicorticis,* Botrytis spp. insbesondere *Botrytis cinerea,* Monilinia spp., Penicillium spp., Coletotrichum spp. hervorgerufen werden. Bei diesen pilzlichen Pathogenen, insbesondere bei *N. malicortcis* und *B. cinerea,* können die besten Ergebnisse erzielt werden und insbesondere eine Ausbreitung der Pflanzenerkrankung bei betroffenen Pflanzen nahezu vollständig unterdrückt werden und auch eine gute Prophylaxe gegen das Auftreten der Pflanzenerkrankungen zur Verfügung gestellt werden.

Die höchsten synergistischen Effekte werden erzielt, wenn, wie dies einer Weiterbildung der Erfindung entspricht, die Zubereitung derart ausgebildet ist, dass *Aureobasidium pullulans* DSM 14940 und DSM 14941 jeweils in einem Zellmengenverhältnis von $1 \times 10^6$ bis $2 \times 10^7$ Zellen/ml Zubereitung, vorzugsweise $1,67 \times 10^6$ bis $3,33 \times 10^6$ Zellen/ml Zubereitung, insbesondere bevorzugt von $2,5 \times 10^6$ Zellen/ml Zubereitung und die chemischen Fungizide Fludioxonil und Cyprodinil in einer Konzentration von vorzugweise 0,0025 g/l Zubereitung bis 1,0 g/l Zubereitung, insbesondere 0,125 g/l Zubereitung bis 0,25 g/l Zubereitung für Fludioxonil bzw. 0,00375 g/l Zubereitung bis 1,0 g/l Zubereitung, insbesondere 0,1875 g/l Zubereitung bis 0,375 g/l Zubereitung für Cyprodinil enthalten sind. Mit einer derartigen Zubereitung können Synergismusfaktoren von bis zu 1,5 erzielt werden.

Eine besonders bevorzugte Verwendung gemäß der Erfindung ist dadurch gekennzeichnet, dass die pilzlichen Pathogene, insbesondere *Botrytis cinerea* sowie *Neofabraea malicoricis* (*Pezicula malicorticis*), gegenüber wenigstens einem chemischen Fungizid, nämlich Fludioxonil eine Resistenz und/oder verringerte Empfindlichkeit bzw. Sensitivität aufweisen. Wenn die pilzlichen Pathogene gegenüber dem wenigstens einen chemischen Fungizid eine Resistenz oder verringerte Empfindlichkeit aufweisen, kann die Zubereitung gemäß der Erfindung eingesetzt werden, da die pilzlichen Pathogene empfindlich gegenüber der eingesetzten Zubereitung sind und die Pathogene erfolgreich unschädlich gemacht werden. Überraschender Weise konnte hierbei insbesondere auch ein chemisches Fungizid zum Einsatz gebracht werden, gegenüber welchem die Resistenz deutlich ausgebildet war. Es wird damit begründet, dass die Pathogene zur Aufrechterhaltung der Resistenz Energie aufwenden müssen, die sie durch den Einsatz der *Aureobasidium pullulans* Stämme DSM 14940 und DSM 14941 und deren antagonistische Wirkung als Nahrungskonkurrent nicht aufbringen können, und so das wenigstens eine chemische Fungizid wieder uneingeschränkt zur Wirkung gebracht werden kann.

Bevorzugt wird hierbei das wenigstens eine chemische Fungizid sowie wenigstens eine die *Aureobasidium pullulans* Stämme DSM 14940 und DSM 14941 enthaltende Mischung in synergistisch wirkenden Mengen eingesetzt. Durch einen derartigen Einsatz kann die Menge an eingesetzten fungiziden Substanzen, und zwar sowohl biologischen als auch chemischen gegenüber den Einzelsubstanzen deutlich herabgesetzt werden, ohne dass die fungizide Wirkung herabgesetzt würde. Auf diese Weise gelingt es einerseits, die Umweltbelastung durch die nicht unbedenklichen chemischen Fungizide, wie Fludioxonil deutlich zu verringern und überdies einen sicheren und zuverlässigen Schutz von Früchten zu erzielen, insbesondere einen Schutz vor pilzlichen Pathogenen, wie N. malicortcis, B. cinerea, Monilinia spp., Penicillium spp., Coletotrichum spp.

Das wenigstens eine chemische Fungizid wird hierbei aus Fludioxonil und wenigstens einem zweiten chemischen Fungizid gewählt aus der Gruppe Cyprodinil, Fenhexamid, Fenpyrazamine und Pyrimethanil oder aus einer Mischung aus Cyprodinil und Fludioxonil in einem Gewichtsverhältnis von 2:1 bis 1:2, vorzugsweise von 2:1 bis 1:1, insbesondere von 1,5:1 gewählt. Indem das chemische Fungizid als Mischung aus Cyprodinil und Fludioxonil in einem Gewichtsverhältnis von 2:1 bis 1:2, insbesondere 1,5:1 eingesetzt wird, kann ein besonders breiter Schutz gegen eine Mehrzahl von pilzlichen Pathogenen erreicht werden.

Die eingesetzte Menge an chemischen Fungiziden kann hierbei noch weiter herabgesetzt werden, wenn die *Aureobasidium pullulans* Stämme DSM 14940 und DSM 14941 in einer einem Zellmengenverhältnis von 2:1 bis 1:2, vorzugsweise 1:1 eingesetzt werden. Das Zellmengenverhältnis bezieht sich insbesondere auf die eingesetzte Zellzahl.

Die Erfindung betrifft schließlich ein Verfahren zur Prophylaxe oder zur Reduktion der Ausbreitung von wenigstens einer durch einen pilzlichen Pathogen ausgelösten Pflanzenerkrankung, welches im Wesentlichen dadurch gekennzeichnet ist, dass

a) wenigstens ein chemisches Fungizid gewählt ist aus Fludioxonil oder einer Kombination enthaltend Fludioxonil und Cyprodinil und
b) eine Mischung enthaltend wenigstens die *Aureobasidium pullulans* Stämme DSM 14940 und DSM 14941

gemeinsam wenigstens einmal auf eine Kulturpflanze aufgebracht werden, wobei a) und b) in synergistisch wirksamen Mengen auf der Kulturpflanze, insbesondere durch Versprühen, Vernebeln oder Beregnen aufgebracht werden.

Mit einem derartigen Verfahren gelingt es pilzliche Pathogene, deren Lebensraum oder die von ihm freizuhaltenden Obst-, Wein- und Gemüsekulturen, insbesondere Äpfel, Weintrauben, Erdbeeren, und Kürbisgewächsen, wie Zucchini und Speisekürbissen durch Aufbringung von wenigstens einem chemischen Fungizid gewählt ist aus Fludioxonil oder einer Kombination enthaltend Fludioxonil und Cyprodinil und den beiden *Aureobasidium pullulans* Stämmen DSM 14940 und DSM 14941 unschädlich zu machen. Vorzugsweise wird das Verfahren so geführt, dass eine Zubereitung durch Versprühen, Vernebeln oder Beregnen aufgebracht wird. Mit einem derartigen Verfahren gelingt es trotz des Einsatzes von geringen Mengen der chemischen Fungizide und biologischen Fungizide eine Wirkzubereitung auf die zu behandelnden Pflanzen, Obst- oder Gemüsesorten, insbesondere Äpfel, aufzubringen, welche sicher und zuverlässig die pilzlichen Pathogene, wie Neofabreaea spp., Botrytis spp., Monilinia spp., Penicillium spp., Coletotrichum spp., insbesondere *N. malicortcis* (*Pezicula malicorticis*) und *B. cinerea* abtötet bzw. deren Ausbreitung eindämmt. Wichtig für das Auftreten der synergistischen Wirkung ist, dass die Komponenten a) und b) gemeinsam und zeitgleich auf der Kulturpflanze vorhanden sind. Es ist theoretisch möglich Komponenten a) und b) getrennt voneinander aufzubringen, oder auch die beiden *Aureobasidium pullulans* Stämme DSM 14940 und DSM 14941 der Komponente b) getrennt voneinander aufzubringen, solange diese auf der Kulturpflanze zur selben Zeit vorliegen bzw. einwirken. Es ist möglich a) und b) bzw. die einzelnen *Aureobasidium pullulans* Stämme DSM 14940 und DSM 14941 in separaten Tankmischungen auf die gewünschte Menge bzw. Konzentration einzustellen und entsprechend der gewünschten Ausbringungsmenge pro Hektar (ha) zeitglich oder zeitlich knapp hintereinander auf die Kulturpflanzen aufzubringen. Der Einfachheit halber und vor allem weil in diesem Fall das gemeinsame Vorliegen der Komponenten der Zubereitung gewährleistet werden kann sowie auch aus Kostengründen ist jedoch die gemeinsame Aufbringung der Komponenten a) und b), d.h. die Komponenten a) und b) liegen in derselben Tankmischung bzw. Zubereitung vor, bevorzugt.

Günstiger Weise wird hierbei das Verfahren so geführt, dass a) und b) gemeinsam in einer Zubereitung gemischt, gelöst oder suspendiert werden und dass die Zubereitung auf die Kulturpflanze aufgebracht wird. Mit einer derartigen Verfahrensführung gelingt es unmittelbar eine gebrauchsfertige Lösung, Suspension oder Mischung herzustellen welche unmittelbar auf zu behandelte Kulturpflanzen aufgebracht werden kann.

Besonders gute Ergebnisse können hierbei dadurch erzielt werden wenn, das Verfahren so geführt wird, dass pro Anwendung jeweils $1 \times 10^{11}$ bis $1 \times 10^{14}$ Zellen/ha, vorzugsweise $1 \times 10^{12}$ bis $2 \times 10^{13}$ Zellen/ha Zubereitung, vorzugsweise $1,67 \times 10^{12}$ bis $3,33 \times 10^{12}$ Zellen/ha Zubereitung, insbesondere bevorzugt von $2,5 \times 10^{12}$ Zellen/ha der einzelnen *Aureobasidium pullulans* Stämme DSM 14940 und DSM 14941 aufgebracht werden. Durch Aufbringung derartiger Zellzahlen der *Aureobasidium pullulans* Stämme DSM 14940 und DSM 14941 konnte eine wirkungsvolle Prophylaxe oder deutliche Reduktion der Ausbreitung von durch pilzliche Pathogene verursachten Pflanzenerkrankungen erreicht werden. In gleicher Weise konnte eine wirkungsvolle Prophylaxe oder deutliche Reduktion der Ausbreitung von durch pilzliche Pathogene verursachten Pflanzenerkrankungen erreicht werden, wenn, wie dies einer Weiterbildung der Erfindung entspricht, das Verfahren so geführt wird, dass als chemisches Fungizid Fludioxonil eingesetzt wird, wobei insbesondere 2,5 g/ha bis 5000 g/ha, vorzugsweise 10 g/ha bis 1000 g/ha, insbesondere 125 g/ha bis 250 g/ha aufgebracht werden oder dass eine Mischung aus Fludioxonil und Cyprodinil eingesetzt wird, wobei insbesondere 3,75 g Cyprodinil/ha bis 5000 g Cyprodinil/ha, vorzugsweise 20 g/ha bis 1000 g/ha, insbesondere 187,5 g/ha bis 375 g/ha aufgebracht werden.

Besonders wirkungsvoll ist, wenn das Verfahren so geführt wird, dass die wenigstens eine Aufbringung des wenigstens einen chemischen Fungizids und der wenigstens beiden *Aureobasidium pullulans* Stämme DSM 14940 und DSM 14941, insbesondere der oben definierten Zubereitung, während der Blütephase der Kulturpflanze erfolgt. Durch eine Aufbringung der Zubereitung während der Blütephase der Kulturpflanze gelingt eine besonders effiziente Prophylaxe oder Reduktion der Ausbreitung von durch pilzliche Pathogene verursachten Pflanzenerkrankungen. Zur Definition der Blütephase der Kulturpflanze wird in international übliche BBCH-Kodierung der Entwicklungsstadien herangezogen wurden, wobei die Blütephasen der Weinrebe hierin der BBCH-Kodierung 53, 55, 57 sowie 60-69 (Lorenz et al., Phänologische Entwicklungsstadien der Weinrebe. Vitic. Enol. Sci. 49, 66-70, 1994) und der Erdbeere hierin der BBCH-Kodierung von 55-59, 60, 61, 65 sowie 67 (Meier et al., Phänologische Entwicklungsstadien des Kernobstes, des Steinobstes, der Johannisbeere und der Erdbeere. Nachrichtenbl. Deut. Pflanzenschutz, 46, 141-153, 1994) entspricht. Ein mehrmaliges gleichzeitiges Aufbringen der Komponenten a) und b) während der Blütephase bzw. während der Blütephase und in den darauffolgenden Entwicklungsstadien, nämlich der Fruchtentwicklung sowie der Fruchtreife können die synergistische Wirkung weiter steigern.

Die Erfindung wird nachfolgend anhand von Ausführungsbeispielen näher erläutert.

Beispiel 1: *Aureobasidium pullulans* Stämme und chemische Fungizide zur Bekämpfung von pilzlichen Pflanzenpathogenen

Die Wirkung der *Aureobasidium pullulans* Stämme AP 241 (DSM 32467), AP 268 (DSM 32468), DSM 14940 und DSM 14941 (einzeln bzw. in Kombination) gemeinsam mit den chemischen Fungiziden Cyprodinil und Fludioxonil (einzeln bzw. in Kombination) gegen die pilzlichen Pflanzenpathogene Botrytis und Neofabreaea wurde in einem Apfeltest bestimmt. Pro Behandlung wurden 12 unbehandelte Äpfel von gleicher Sorte mit einheitlichem Reifegrad und Größe verwendet. Vor dem Test wurden die Äpfel für drei Minuten in 70 % Ethanol oberflächensterilisiert und unter der Sterilbank getrocknet. Pro Apfel wurden in der äquatorialen Ebene zwischen Calyx und Stiel mit einer sterilen Pipettenspitze vier Wunden mit einem Wunddurchmesser von 5 mm in gleichem Abstand gesetzt. Pro Apfel wurden unmittelbar nach Herstellung der vier künstlichen Wunden, jeweils 10 μl von unterschiedlichen Testlösungen (Zubereitungen) aufgebracht. Im Anschluss wurden die Äpfel gelagert. Um die Wunden bildete sich eine Faulstelle, deren Durchmesser ein Maß für die Ausbreitung der Infektion des Pathogens darstellt. Für die Auswertung wurde der Durchmesser der Einstiche von je 0,5 cm vom Faulstellendurchmesser abgezogen.

Die 4 Testlösungen die in jeweils eine der 4 Wunden pro Apfel gegeben wurden, waren:

1. Pathogen (negative Kontrolle)
2. Pathogen und Aureobasidium pullulans (einzeln bzw. in Kombination)
3. Pathogen und chemisches Fungizid (einzeln bzw. in Kombination)
4. Pathogen und Aureobasidium pullulans (einzeln bzw. in Kombination) und chemisches Fungizid (einzeln bzw. in Kombination)

Aus den Faulstellendurchmessern, die am Ende der Lagerung gemessen wurden, wurden für die Testlösungen 2 bis 4 die Wirkungsgrade der Hemmung des Pathogens errechnet. Dieser Wirkungsgrad entspricht der prozentuellen Abnahme des Faulstellendurchmessers im Vergleich zur jeweiligen negativen Kontrolle. Wäre z.B. der Faulstellendurchmesser von Testlösung 1: 5 cm und der der Testlösungen 2, 3 bzw. 4 je 1 cm so wäre der Wirkungsgrad von Testlösung 2 bis 4 jeweils 80 %. Der Wirkungsgrad der negativen Kontrolle ist per Definition Null.

Aus den Wirkungsgraden (WG) der Einzelkomponenten in den Testlösungen 2 und 3 wurden nach der Colby-Formel (S.R. Colby "Calculating synergistic and antagonistic responses of herbicide combinations", Weeds 15, Seite 20 bis 22, 1967) Erwartungswerte (E) der Wirkung der Kombination aus Aureobasidium und chemischen Fungizid (Testlösung 4) nach folgender Formel berechnet werden.

$$E = WG\ X + (WG\ Y\ /\ 100) \times (100 - WG\ X)$$

E          Erwartungswert
WG X      Wirkungsgrad Einzelkomponente X
WG Y      Wirkungsgrad Einzelkomponente Y

ist der gemessene Wirkungsgrad der Kombination der *Aureobasidium pullulans* Stämme und chemischen Fungizid (Testlösung 4) größer als der errechnete Erwartungswert (E), spricht man von einem synergistischen Effekt (die Einzelkomponenten verstärken sich gegenseitig überadditiv). Der Synergismusfaktor ist ein Maß für den synergistischen Effekt und wurde berechnet als Quotient aus Wirkungsgrad der Kombination der Substanzen bzw. Komponenten (Testlösung 4) und dem errechneten Erwartungswert (E). ist der Synergismusfaktor größer gleich 1,1 ist die Wirkung der Kombination synergistisch gegenüber der Wirkung der Einzelkomponenten. Wird in diesem Dokument von "Synergismus" oder "synergistisch wirkend" oder "Synergistische Wirkung" oder dergleichen gesprochen, bezieht sich dies immer auf Kombinationen mit einem Synergismusfaktor von größer gleich 1,1, im Vergleich zu den Einzelkomponenten der Kombination. Pro Versuchsanordnung, bestehend auf den Testlösungen 1 bis 4, wurden 12 Replikate (12 Äpfel) untersucht.

Getestete pilzliche Pflanzenpathogene:

Botrytis spp.:

Botrytis cinerea Bc97 (DSM 32469)
Dieser Stamm besitzt Resistenzen gegen die chemischen Fungizide Strobilurin, Boscalid und Cyprodinil sowie eine verringerte Sensitivität gegen Fludioxonil.
Botrytis cinerea 12/4 (DSM 32486)
Dieser Stamm besitzt gegenüber chemischen Fungiziden keine bekannten verringerten Sensitivitäten bzw. Resistenzen.
Neofabreaea spp.: *Pezicula malicorticis* 160622 (DSM 62715)

Pathogenlösungen:

Zur Herstellung der Pathogenlösungen wurden von mit Pathogen bei 20 °C bewachsenen ME-Nähragarplatten (ME-Agar: 30 g Malzextrakt, 15 g Agar, 5 g Pepton auf 1000 ml destilliertes Wasser) Konidien von einer Fläche von ca. 1-2 cm mal 2 cm abgenommen und

in 10 ml Wasser mit einem Douncer suspendiert. Danach wurde die Konidienkonzentration in der Thomakammer mikroskopisch ausgezählt und mit Wasser auf die gewünschte Konzentration eingestellt.

Botrytis

Endkonzentration der Konidien in der Testlösung: $2 \times 10^5$ Konidien/ml

Konzentration der Konidien in der $2 \times$ konzentrierten Pathogenlösung: $4 \times 10^5$ Konidien/ml

Neofabreaea

Endkonzentration der Konidien in der Testlösung: $1 \times 10^6$ Konidien/ml

Konzentration der Konidien in der $2 \times$ konzentrierten Pathogenlösung: $2 \times 10^6$ Konidien/ml

*Aureobasidium pullulans* Stämme:

DSM 14940

DSM 14941

AP 241 (DSM 32467) isolierter *Aureobasidium pullulans* Stamm

AP 268 (DSM 32468) isolierter *Aureobasidium pullulans* Stamm

Aureobasidium pullulans Konzentrationen:

Die *Aureobasidium pullulans* Stämme DSM 14940, DSM 14941, AP 241 und AP 268 wurden auf Nähragarplatten (YM-Agar: 3 g Hefeextrakt, 3 g Malzextrakt, 5 g Pepton, 10 g Glukose und 20 g Agar auf 1000 ml destilliertes Wasser) bei 27 °C kultiviert und die Zellen wurden mit 0,6 %-iger NaCl Lösung geerntet. Die Konzentration der Hefezellen wurde in der Thomakzählkammer mikroskopisch ausgezählt und mit Wasser auf die gewünschte Konzentration eingestellt. Untersuchungen der Zellviabilität sowie der koloniebildenden Einheiten ergaben, dass >99,9% der Zellen viabel sind und Kolonien ausbilden können. Somit stehen die Angabe der Zellen insbesondere der Zellen/ml hierin auch synonym für Koloniebildende Einheiten (KBE) und insbesondere für KBU/ml.

$2 \times$ konzentrierte Aureobasidiumlösung: $1 \times 10^7$ Zellen/ml

$4 \times$ konzentrierte Aureobasidiumlösung: $2 \times 10^7$ Zellen/ml

Endkonzentrationen in der Testlösung (Zubereitung):

DSM 14940, DSM 14941, AP 241 und AP 268: $5 \times 10^6$ Zellen/ml

AP 1: 1:1 Mischung aus DSM 14940 und DSM 14941: $2,5 \times 10^6$ Zellen/ml DSM 14940 und $2,5 \times 10^6$ Zellen/ml DSM 14941

AP 2: 1:1 Mischung aus DSM 14940 und AP 241: $2,5 \times 10^6$ Zellen/ml DSM 14940 und $2,5 \times 10^6$ Zellen/ml AP 241

AP 3: 1:1 Mischung aus DSM 14940 und AP 268: $2,5 \times 10^6$ Zellen/ml DSM 14940 und $2,5 \times 10^6$ Zellen/ml AP 268

AP 4: 1:1 Mischung aus DSM 14941 und AP 241: $2,5 \times 10^6$ Zellen/ml DSM 14941 und $2,5 \times 10^6$ Zellen/ml AP 241

AP 5: 1:1 Mischung aus DSM 14941 und AP 268: $2,5 \times 10^6$ Zellen/ml DSM 14941 und $2,5 \times 10^6$ Zellen/ml AP 268

AP 6: 2:1 Mischung aus DSM 14940 und DSM 14941 ($3,33 \times 10^6$ Zellen/ml DSM 14940 und $1,67 \times 10^6$ Zellen/ml DSM 14941)

AP 7: 1:2 Mischung aus DSM 14940 und DSM 14941 ($1,67 \times 10^6$ Zellen/ml DSM 14940 und $3,33 \times 10^6$ Zellen/ml DSM 14941)

Chemische Fungizide:

Es wurden folgende chemische Fungizide mit in den in der Tabelle angegeben Konzentration eingesetzt:

| # | Konzentration des Wirkstoffs des chemische Fungizids in der Testlösung bzw. Zubereitung | 2 × konzentrierte chemische Fungizidlösung | 4 × konzentrierte chemische Fungizidlösung |
|---|---|---|---|
| A | 0,01875 % (w/v) Cyprodinil (= 0,1875 g/l Zubereitung)<br><br>0,01250 % (w/v) Fludioxonil (= 0,125 g/l Zubereitung) | 0,0375 % (w/v) Cyprodinil<br>0,0250 % (w/v) Fludioxonil | 0,075 % (w/v) Cyprodinil<br><br>0,050 % (w/v) Fludioxonil |
| B | 0,01250 % (w/v) Fludioxonil (= 0,125 g/l Zubereitung) | 0,0250 % (w/v) Fludioxonil | 0,050 % (w/v) Fludioxonil |
| C | 0,01875 % (w/v) Cyprodinil (= 0,1875 g/l Zubereitung) | 0,0375 % (w/v) Cyprodinil | 0,075 % (w/v) Cyprodinil |

Testlösungen pro Wunde:

| Wundel Testlösung | Ansatz | Zusammensetzung der in die Apfelwunde aufgetragenen Testlösung |
|---|---|---|
| 1 | Pathogenkontrolle | 5 µl 2 × konzentrierte Pathogenlösung<br>5 µl Wasser |
| 2 | Pathogen + Aureobasidium | 5 µl 2 × konzentrierte Pathogenlösung<br>5 µl 2 × konzentrierte Aureobasidiumlösung |
| 3 | Pathogen + chemisches Fungizid | 5 µl 2 × konzentrierte Pathogenlösung<br>5 µl 2 × konzentrierte chemische Fungizidlösung |
| 4 | Pathogen + Aureobasidium + chemisches Fungizid | 5 µl 2 × konzentrierte Pathogenlösung<br>2,5 µl 4 × konzentrierte Aureobasidiumlösung<br>2,5 µl 4 × konzentrierte chemische Fungizidlösung |

Ergebnisse

Botrytis cinerea Bc97
Testsystem: Äpfel der Sorte Jonagold Honsel
Lagerungstemperatur: 20 °C; Lagerdauer: 6 Tage
Pathogen: *Botrytis cinerea* Bc97
*Aureobasidium pullulans* Stämme: DSM 14940, DSM 14941, AP 241, AP 268
Chemische Fungizide: Fludioxonil, Cyprodinil

| | Aureobasidium Stamm | DSM 14940 | DSM 14941 | AP 241 | AP 268 |
|---|---|---|---|---|---|
| | Chemisches Fungizid (w/v) | 0,125 g/l Fludioxonil | | | |
| Mittlerer Durchmesser Faulstellen (cm) | Pathogenkontrolle | 3,18 | 3,44 | 3,03 | 2,62 |
| | Pathogen + Aureobasidium | 3,03 | 2,65 | 2,80 | 2,45 |
| | Pathogen + chemisches Fungizid | 0,68 | 0,28 | 0,48 | 0,53 |
| | Pathogen + Aureobasidium + chemisches Fungizid | 0,78 | 0,40 | 0,42 | 0,45 |
| Wirkungsgrad | Pathogenkontrolle | 0,0 | 0,0 | 0,0 | 0,0 |
| | Pathogen + Aureobasidium | 4,7 | 23,0 | 7,7 | 6,4 |
| | Pathogen + chemisches Fungizid | 78,8 | 91,8 | 84,3 | 79,9 |
| | Pathogen + Aureobasidium + chemisches Fungizid | 75,7 | 88,4 | 86,3 | 82,8 |
| Erwartungswert (E) | Pathogen + Aureobasidium + chemisches Fungizid | 79,8 | 93,7 | 85,5 | 81,2 |
| Synergismusfaktor | | 1,0 | 0,9 | 1,0 | 1,0 |

| | Aureobasidium Stamm | DSM 14940 | DSM 14941 | AP 241 | AP 268 |
|---|---|---|---|---|---|
| | Chemisches Fungizid (w/v) | 0,1875 g/l Cyprodinil | | | |
| Mittlerer Durchmesser Faulstellen (cm) | Pathogenkontrolle | 3,41 | 3,56 | 2,95 | 3,13 |
| | Pathogen + Aureobasidium | 3,30 | 3,45 | 2,88 | 2,96 |
| | Pathogen + chemisches Fungizid | 2,80 | 3,14 | 2,21 | 2,52 |
| | Pathogen + Aureobasidium + chemisches Fungizid | 2,73 | 3,04 | 2,18 | 2,49 |
| Wirkungsgrad | Pathogenkontrolle | 0,0 | 0,0 | 0,0 | 0,0 |
| | Pathogen + Aureobasidium | 3,2 | 3,1 | 2,4 | 5,4 |
| | Pathogen + chemisches Fungizid | 17,9 | 11,8 | 25,1 | 19,5 |
| | Pathogen + Aureobasidium + chemisches Fungizid | 19,9 | 14,6 | 26,1 | 20,4 |
| Erwartungswert (E) | Pathogen + Aureobasidium + chemisches Fungizid | 20,5 | 14,5 | 26,9 | 23,9 |
| Synergismusfaktor | | 1,0 | 1,0 | 1,0 | 0,9 |

| | Aureobasidium Stamm | DSM 14940 | DSM 14941 | AP 241 | AP 268 |
|---|---|---|---|---|---|
| | Chemisches Fungizid (w/v) | 0,1875 g/l Cyprodinil 0,125 g/l Fludioxonil | | | |
| Mittlerer Durchmesser Faulstellen (cm) | Pathogenkontrolle | 3,65 | 3,56 | 2,95 | 3,13 |
| | Pathogen + Aureobasidium | 1,45 | 1,77 | 1,65 | 1,57 |
| | Pathogen + chemisches Fungizid | 1,73 | 1,93 | 1,64 | 1,82 |
| | Pathogen + Aureobasidium + chemisches Fungizid | 1,54 | 1,38 | 1,2 6 | 1,25 |
| Wirkungsgrad | Pathogenkontrolle | 0,0 | 0,0 | 0,0 | 0,0 |
| | Pathogen + Aureobasidium | 60,3 | 50,3 | 44,1 | 49,8 |
| | Pathogen + chemisches Fungizid | 52,6 | 45,8 | 44,4 | 41,9 |
| | Pathogen + Aureobasidium + chemisches Fungizid | 57,8 | 61,2 | 57,3 | 60,1 |
| Erwartungswert (E) | Pathogen + Aureobasidium + chemisches Fungizid | 81,2 | 73,0 | 68,9 | 70,8 |
| Synergismusfaktor | | 0,7 | 0,8 | 0,8 | 0,8 |

Aus oben stehenden Tabellen ist klar erkennbar, dass die Anwendungen der einzelnen *Aureobasidium pullulans* Stämme gemeinsam mit den chemischen Fungiziden Fludioxonil bzw. Cyprodinil als auch gemeinsam mit der der Mischung aus Fludioxonil und Cyprodinil keine synergistische Wirkung besitzen.

| | Aureobasidium Stamm | AP1: DSM 14940 + DSM 14941 | | |
|---|---|---|---|---|
| | Chemisches Fungizid (w/v) | 0,1875 g/l Cyprodinil 0,125 g/l Fludioxonil | 0,125 g/l Fludioxonil | 0,1875 g/l Cyprodinil |
| Mittlerer Durchmesser Faulstellen (cm) | Pathogenkontrolle | 4,17 | 4,21 | 4,17 |
| | Pathogen + Aureobasidium | 1,42 | 1,50 | 2,33 |
| | Pathogen + chemisches Fungizid | 1,83 | 1,17 | 3,63 |
| | Pathogen + Aureobasidium + chemisches Fungizid | 0,33 | 0,08 | 1,83 |
| Wirkungsgrad | Pathoenkontrolle | 0,0 | 0,0 | 0,0 |
| | Pathogen + Aureobasidium | 66,0 | 64,4 | 44,0 |
| | Pathogen + chemisches Fungizid | 56,0 | 72,3 | 13,0 |
| | Pathogen + Aureobasidium + chemisches Fungizid | 92,0 | 98,0 | 56,0 |
| Erwartungswert (E) | Pathogen + Aureobasidium + chemisches Fungizid | 85,0 | 90,1 | 51,3 |
| Synergismusfaktor | | 1,1 | 1,1 | 1,1 |

| | Aureobasidium Stamm | AP 6: DSM 14940 + DSM 14941 | | |
|---|---|---|---|---|
| | Chemisches Fungizid (w/v) | 0,1875 g/l Cyprodinil 0,125 g/l Fludioxonil | 0,125 g/l Fludioxonil | 0,1875 g/l Cyprodinil |
| Mittlerer Durchmesser Faulstellen (cm) | Pathogenkontrolle | 4,02 | 4,11 | 3,89 |
| | Pathogen + Aureobasidium | 1,64 | 1,83 | 2,29 |
| | Pathogen + chemisches Fungizid | 1,74 | 1,62 | 3,51 |
| | Pathogen + Aureobasidium + chemisches Fungizid | 0,41 | 0,05 | 1,67 |
| Wirkungsgrad | Pathogenkontrolle | 0,0 | 0,0 | 0,0 |
| | Pathogen + Aureobasidium | 59,2 | 55,5 | 41,1 |
| | Pathogen + chemisches Fungizid | 56,7 | 60,6 | 9,8 |
| | Pathogen + Aureobasidium + chemisches Fungizid | 89,8 | 98,8 | 57,1 |
| Erwartungswert (E) | Pathogen + Aureobasidium + chemisches Fungizid | 82,3 | 82,4 | 46,9 |
| Synergismusfaktor | | 1,1 | 1,2 | 1,2 |

| | Aureobasidium Stamm | AP 7: DSM 14940 + DSM 14941 | | |
|---|---|---|---|---|
| | Chemisches Fungizid (w/v) | 0,1875 g/l Cyprodinil 0,125 g/l Fludioxonil | 0,125 g/l Fludioxonil | 0,1875 g/l Cyprodinil |
| Mittlerer Durchmesser Faulstellen (cm) | Pathogenkontrolle | 3,68 | 3,74 | 4,00 |
| | Pathogen + Aureobasidium | 1,78 | 1,96 | 2,51 |
| | Pathogen + chemisches Fungizid | 1,83 | 2,04 | 3,38 |
| | Pathogen + Aureobasidium + chemisches Fungizid | 0,32 | 0,54 | 1,78 |
| Wirkungsgrad | Pathogenkontrolle | 0,0 | 0,0 | 0,0 |
| | Pathogen + Aureobasidium | 51,6 | 47,6 | 37,3 |
| | Pathogen + chemisches Fungizid | 50,3 | 45,5 | 15,5 |
| | Pathogen + Aureobasidium + chemisches Fungizid | 91,3 | 85,6 | 55,5 |
| Erwartungswert (E) | Pathogen + Aureobasidium + chemisches Fungizid | 75,9 | 71,4 | 47,0 |
| Synergismusfaktor | | 1,2 | 1,2 | 1,2 |

Aus oben stehender Tabelle ist klar erkennbar, dass die Anwendung von Mischungen der beiden Aureobasidium Stämme DSM 14940 und DSM 14941 gemeinsam mit den chemischen Fungiziden Cyprodinil bzw. Fludioxonil als auch gemeinsam mit der Mischung aus Cyprodinil und Fludioxonil deutliche synergistische Wirkungen besitzen. Der Wirkungsgrad der Zubereitungen aus DSM 14940 und DSM 14941 mit den chemisches Fungizid(en) (Testgruppen 4) liegen jeweils deutlich über dem errechneten Erwartungswert (E). Der Synergismusfaktor ist größer gleich 1,1.

In der AP 1 sowie in der AP 6 Gruppe jeweils gemeinsam mit Fludioxonil kam zu nahezu keiner Fäulnisbildung (Durchmesser der Faulstelle von 0,08 cm bzw. 0,05 cm), was die sehr gute prophylaktische Wirkung der erfinderischen Zubereitungen bestätigt. Bei den restlichen Versuchen der Gruppen AP 1, AP 6 und AP 7 zeigten sich deutliche Reduktionen der Fäulnisbildungen, was die Reduktion der Ausbreitung der Pilzerkrankung durch die erfinderischen Zubereitungen zeigt.

| | Aureobasidium Stamm | AP 2: DSM 14940+ AP 241 | AP 3: DSM 14940+ AP 268 | AP 4: DSM 14941+ AP 241 | AP 5: DSM 14941+ AP 268 |
|---|---|---|---|---|---|
| | Chemisches Fungizid (w/v) | 0,125 g/l Fludioxonil | | | |
| Mittlerer Durchmesser Faulstellen (cm) | Pathogenkontrolle | 3,13 | 3,18 | 2,99 | 2,98 |
| | Pathogen + Aureobasidium | 2,97 | 2,83 | 2,73 | 2,70 |
| | Pathogen + chemisches Fungizid | 1,61 | 1,53 | 1,53 | 1,34 |
| | Pathogen + Aureobasidium + chemisches Fungizid | 1,54 | 1,47 | 1,62 | 1,52 |
| Wirkungsgrad | Pathogenkontrolle | 0,0 | 0,0 | 0,0 | 0,0 |
| | Pathogen + Aureobasidium | 5,1 | 11,0 | 8,9 | 9,5 |
| | Pathogen + chemisches Fungizid | 48,5 | 51,8 | 48,9 | 55,1 |
| | Pathogen + Aureobasidium + chemisches Fungizid | 50,7 | 53,7 | 45,8 | 49,1 |
| Erwartung s-wert (E) | Pathogen + Aureobasidium + chemisches Fungizid | 51,1 | 57,1 | 53,4 | 59,3 |
| Synergismusfaktor | | 1,0 | 0,9 | 0,9 | 0,8 |

Die synergistische Wirkung von Mischungen von AP 1, AP 6 und AP 7 mit den beiden chemischen Fungiziden Cyprodinil und Fludioxonil gegenüber dem Pathogen *Botrytis cinerea* Bc97 wurde analog zu obigen Versuchen auch mit unterschiedlichen Konzentrationen an chemischem Fungizid bestätigt.

Für Cyprodinil konnte für allen Konzentrationen, nämlich 0,02 g/l; 0,06 g/l; 0,125 g/l; 0,375 g/l; 0,5 g/l und 1,0 g/l Zubereitung die synergistische Wirkung bestätigt werden (Synergismusfaktor größer gleich 1,1), wobei für die Konzentrationen 0,125 g/l und 0,375 g/l jeweils der höchste Synergismusfaktor bestimmt wurde.

Für Fludioxonil konnte für alle Konzentrationen, nämlich 0,01 g/l; 0,05 g/l; 0,125 g/l; 0,25 g/L; 0,5 g/l und 1,0 g/l die synergistische Wirkung bestätigt werden (Synergismusfaktor größer gleich 1,1), wobei für die Konzentrationen 0,125 g/l und 0,25 g/l jeweils der höchste Synergismusfaktor bestimmt wurde.

Botrytis cinerea 12/4
Testsystem: Äpfel der Sorte Jonagold Honsel
Lagerungstemperatur: 20 °C; Lagerdauer: 7 Tage
Pathogen: Botrytis cinerea 12/4 (keine Sensitivität bzw. Resistenz gegenüber Fludioxonil und/oder Cyprodinil)
Aureobasidium: DSM 14940, DSM 14941 und AP 1
Chemische Fungizide: Fludioxonil, Cyprodinil; es wurde nur 1/50 der oben angegebenen Menge an chemischem Fungizid eingesetzt.

| | Aureobasidium Stamm | DSM 14940 | DSM 14941 | AP 1: DSM 14940 + DSM 14941 |
|---|---|---|---|---|
| | Chemisches Fungizid (w/v) | 0,000375% Cyprodinil (= 0,00375 g/l Zubereitung) 0,00025% Fludioxonil (= 0,0025 g/l Zubereitung) | | |
| Mittlerer Durchmesser Faulstellen (cm) | Pathogenkontrolle | 3,60 | 3,57 | 3,63 |
| | Pathogen + Aureobasidium | 1,88 | 2,22 | 1,64 |
| | Pathogen + chemisches Fungizid | 1,45 | 1,67 | 1,38 |
| | Pathogen + Aureobasidium + chemisches Fungizid | 1,53 | 1,58 | 0,03 |
| Wirkungsgrad | Pathogenkontrolle | 0,0 | 0,0 | 0,0 |
| | Pathogen + Aureobasidium | 47,8 | 37,8 | 54,8 |
| | Pathogen + chemisches Fungizid | 59,7 | 53,2 | 62,0 |
| | Pathogen + Aureobasidium + chemisches Fungizid | 57,5 | 55,7 | 99,1 |
| Erwartungswert (E) | Pathogen + Aureobasidium + chemisches Fungizid | 79,0 | 70,9 | 82,8 |
| Synergismusfakt or | | 0,7 | 0,8 | 1,2 |

Aus oben stehender Tabelle ist klar erkennbar, dass nur die Anwendung der Mischung aus beiden Aureobasidium Stämme DSM 14940 und DSM 14941 gemeinsam mit den chemischen Fungiziden Cyprodinil und Fludioxonil (AP 1 Gruppe) eine synergistische Wirkung besitzt. Der Wirkungsgrad der Testgruppe 4 (Mischung der Aureobasidium Stämme + chemisches Fungizid) liegt hierfür deutlich über dem errechneten Erwartungswert (E). Der Synergismusfaktor ist größer gleich 1,1. Es kam zu nahezu keiner Fäulnisbildung (Durchmesser der Faulstelle von 0,03 cm), was die sehr gute prophylaktische Wirkung der erfinderischen Zubereitung bestätigt.

Die synergistische Wirkung von Mischungen von AP 1, AP 6 und AP 7 mit den beiden chemischen Fungiziden Cyprodinil und Fludioxonil gegenüber dem Pathogen *Botrytis cinerea* 12/4 wurde analog zu obigen Versuchen auch mit unterschiedlichen Konzentrationen an chemischem Fungizid bestätigt.

Für Cyprodinil konnte für allen Konzentrationen, nämlich 0,001 g/l; 0,00375 g/l; 0,005 g/l; 0,01 g/l; 0,05 g/l; 0,1 g/l und 0,1875 g/l Zubereitung die synergistische Wirkung bestätigt werden (Synergismusfaktor größer gleich 1,1).

Für Fludioxonil konnte für alle Konzentrationen, nämlich 0,001 g/l; 0,0025 g/l; 0,005 g/l; 0,01 g/l; 0,05 g/l; 0,1 g/l und 0,125 g/l Zubereitung die synergistische Wirkung bestätigt werden (Synergismusfaktor größer gleich 1,1).

Da die eingesetzten Mengen an chemischen Fungiziden vom Pathogen selbst, insbesondere aber von seinen Resistenzen bzw. verminderten Sensitivitäten gegenüber den einzelnen chemischen Fungiziden abhängt, ist davon auszugehen, dass für manche Pathogene auch eine höhere oder geringere Einsatzmenge des chemischen Fungizids ausreicht, um mit der *Aureobasidium pullulans* Mischung aus DSM 14940 und DSM 14941 synergistisch zu wirken.

Neofabraea spp:

Testsystem: Äpfel der Sorte Eistar Fuchshof
Lagerungstemperatur: 20 °C; Lagerdauer: 16 Tage
Pathogen: Pezicula malicorticis 160622 (DSM 62715)
Aureobasidium: DSM 14940, DSM 14941 und AP 1 (anders als oben angegeben war hier die Gesamtkonzentration der eingesetzten *Aurobasidium pullulans* Stämme bei $1 \times 10^7$ Zellen/ml)
Chemische Fungizide: Fludioxonil, Cyprodinil

| Aureobasidium Stamm | | AP 1 (DSM 14940 + DSM 14941) | | |
|---|---|---|---|---|
| Chemisches Fungizid (w/v) | | 0,1875 g/l Cyprodinil 0,125 g/l Fludioxonil | 0,125 g/l Fludioxonil | 0,1875 g/l Cyprodinil |
| Mittlerer Durchmesser Faulstellen (cm) | Pathogenkontrolle | 1,90 | 1,84 | 1,94 |
| | Pathogen + Aureobasidium | 1,30 | 1,23 | 1,44 |
| | Pathogen + chemisches Fungizid | 0,94 | 1,91 | 1,61 |
| | Pathogen + Aureobasidium + chemisches Fungizid | 0,33 | 1,09 | 0,83 |
| Wirkungsgrad | Pathogenkontrolle | 0,0 | 0,0 | 0,0 |
| | Pathogen + Aureobasidium | 31,6 | 33,2 | 25,8 |
| | Pathogen + chemisches Fungizid | 50,5 | -3,8 | 17,0 |
| | Pathogen + Aureobasidium + chemisches Fungizid | 82,6 | 40,8 | 57,2 |
| Erwartungswert (E) | Pathogen + Aureobasidium + chemisches Fungizid | 66,8 | 30,6 | 38,4 |
| Synergismusfaktor | | 1,1 | 1,3 | 1,5 |

Aus oben stehender Tabelle ist klar erkennbar, dass die Anwendung der beiden Aureobasidium Stämme DSM 14940 und DSM 14941 gemeinsam mit den chemischen Fungiziden Cyprodinil und Fludioxonil eine deutliche synergistische Wirkung besitzen. Der Wirkungsgrad der Pathogen + Aureobasidium + chemisches Fungizid (Testgruppe 4) liegt jeweils deutlich über dem errechneten Erwartungswert (E). Der Synergismusfaktor ist größer gleich 1,1.

Weiterhin wurden auch die Einzelstämme in einer Konzentration von $1 \times 10^7$ Zellen/ml in der Testlösung mit den chemischen Fungiziden Cyprodinil (0,01875 % (w/v) = 0,1875 g Cyprodinil pro I Zubereitung) und Fludioxonil (0,01250 % (w/v) = 0,125 g Fludioxonil pro I Zubereitung) einzeln und in Kombination (0,01875% (w/v) Cyprodinil; 0,01250 % (w/v) Fludioxonil) getestet. Der Synergismusfaktor war dabei kleiner gleich 1,0 und somit ist die Anwendung der chemischen Fungizide mit nur einem *Aureobasidium pullulans* Stamm nicht synergistisch.

Die synergistische Wirkung von Mischungen von AP 1, AP 6 und AP 7 mit den beiden chemischen Fungiziden Cyprodinil und Fludioxonil gegenüber dem Pathogen *Pezicula malicorticis* 160622 wurde analog zu obigen Versuchen auch mit unterschiedlichen Konzentrationen an chemischem Fungizid bestätigt.

Für Cyprodinil konnte für allen Konzentrationen, nämlich 0,02 g/l; 0,06 g/l; 0,125 g/l; 0,375 g/l; 0,5 g/l und 1,0 g/l Zubereitung die synergistische Wirkung bestätigt werden (Synergismusfaktor größer gleich 1,1), wobei für die Konzentrationen 0,125 g/l und 0,375 g/l jeweils der höchste Synergismusfaktor bestimmt wurde.

Für Fludioxonil konnte für alle Konzentrationen, nämlich 0,01 g/l; 0,05 g/l; 0,125 g/l; 0,25 g/l; 0,5 g/l und 1,0 g/l die synergistische Wirkung bestätigt werden (Synergismusfaktor größer gleich 1,1), wobei für die Konzentrationen 0,125 g/l und 0,25 g/l jeweils der höchste Synergismusfaktor bestimmt wurde.

Beispiel 2: Feldversuche

in Freilandversuchen (Feldversuchen) konnte bei der Bekämpfung von Botrytis spp., im Wesentlichen von Botrytis cinerea, beim Wein (Tafeltrauben) und bei Erdbeeren gezeigt werden, dass die Tanksprühmischung (Zubereitung) von *Aureobasidium pullulans* DSM 14941 und DSM 14940 gemeinsam mit den chemischen Wirkstoffen Cyprodinil und Fludioxonil den Wirkungsgrad auf den Pathogen synergistisch steigern konnten.

Testsystem A: Tafeltrauben (Vitis vinifera)

Ort: Italien
Dauer: 11. September bis 11. November 2014

Anzahl / Art der Aufbringung der Tanksprühmischungen: 5 identische Behandlungen zu je 1.000 l/ha. Die Tankmischungen wurden durch Sprühen aufgebracht. Die Sprühanwendung erfolgte jeweils entsprechend der BBCH-Kodierung der phänologischen Entwicklungsstadien der Weinrebe (Lorenz et al., Phänologische Entwicklungsstadien der Weinrebe. Vitic. Enol. Sci. 49, 66-70, 1994) in folgenden Entwicklungsstadien:

BBCH 53: «Gescheine» (Infloreszenzen) deutlich sichtbar
Testsystem B: Erdbeeren (Fragaria ananassa)
Ort: Österreich
Dauer: 9. Mai bis 10. Juni 2016

Anzahl / Art der Aufbringung der Tankmischungen: 6 identische Behandlungen mit je 1.000 l/ha. Die Tankmischungen wurden durch Sprühen aufgebracht.

Die Sprühanwendung erfolgte jeweils entsprechend der BBCH-Kodierung der phänologischen Entwicklungsstadien der Erdbeere (Meier et al., Phänologische Entwicklungsstadien des Kernobstes, des Steinobstes, der Johannisbeere und der Erdbeere. Nachrichtenbl. Deut. Pflanzenschutz., 46, 141-153, 1994) in folgenden Entwicklungsstadien:
BBCH 55: Erste Blütenanlagen werden am Rosettengrund sichtbar
Es wurden dabei in den jeweiligen Versuchsgruppen folgende Sprühmischung direkt auf die Feldfrüchte aufgetragen:
Gruppe 1: unbehandelte Kontrolle
Gruppe 2: mit Cyprodinil und Fludioxonil behandelt; Konzentrationen in der Tankmischung: 0,1875 gll Cyprodinil und 0,125 g/l Fludioxonil. Somit wurden pro Hektar (ha) 187,5 g Cyprodinil und 125 g Fludioxonil auf die Pflanzenkulturen aufgebracht.
Gruppe 3: mit AP 1 behandelt; *Aureobasidium pullulans* Konzentrationen in der Tankmischung: $2,5 \times 10^6$ Zellen/ml DSM 14940 und $2,5 \times 10^6$ Zellen/ml DSM 14941. Somit wurden pro Hektar (ha) $2,5 \times 10^{12}$ Zellen DSM 14940 und $2,5 \times 10^{12}$ Zellen DSM 14941 aufgebracht.
Gruppe 4: mit Cyprodinil und Fludioxonil sowie mit AP 1 behandelt; Konzentrationen in der Tankmischung (= Zubereitung): 0,1875 g/l Cyprodinil und 0,125 g/l Fludioxonil sowie $2,5 \times 10^6$ Zellen/ml DSM 14940 und $2,5 \times 10^6$ Zellen/ml DSM 14941. Somit wurden pro Hektar (ha) 187,5 g Cyprodinil und 125 g Fludioxonil auf die Pflanzenkulturen sowie $2,5 \times 10^{12}$ Zellen DSM 14940 und $2,5 \times 10^{12}$ Zellen DSM 14941 aufgebracht.

| Synergie von AP 1+ Mischung Cyprodinil und Fludioxonil | Feldfrucht | Tafeltraube | Erdbeere |
|---|---|---|---|
| Anteil der vom Pathogen befallenen Früchte [%] | Gruppe 1 | 27,30 | 62,30 |
| | Gruppe 2 | 9,80 | 27,60 |
| | Gruppe 3 | 15,10 | 45,80 |
| | Gruppe 4 | 2,30 | 10,10 |
| Wirkungsgrad | Gruppe 1 | 0,0 | 0,0 |
| | Gruppe 2 | 64,1 | 55,7 |
| | Gruppe 3 | 44,7 | 26,5 |
| | Gruppe 4 | 91,6 | 83,8 |
| Erwartungswert (E) | Gruppe 4 | 80,1 | 67,4 |
| Synergismusfaktor | | 1,1 | 1,2 |

Berechnung des Wirkungsgrads: z.B. für Gruppe 4: Wirkungsgrad = [1 - (2,30/27,30)] * 100
Aus obiger Tabelle die synergistische Reduktion der Ausbreitung des Pathogens durch die gemeinsame Anwendung der AP 1 Mischung mit den chemischen Fungiziden deutlich zu erkennen. Der Synergismusfaktor ist jeweils größer gleich 1,1.
Analog zur obigen Tabelle wurden in den Feldversuchen (Tafeltraube und Erdbeere) auch weitere Gruppen folgende Gruppen getestet:

Gruppe 5: mit Cyprodinil behandelt; Konzentration von Cyprodinil in der Tankmischung: 0,1875 g/l; Ausbringung: 1.000 l/ha

Gruppe 6: mit Fludioxonil behandelt; Konzentration von Fludioxonil in der Tankmischung: 0,125 g/l; Ausbringung:

1.000 l/ha

Gruppe 7: mit Cyprodinil sowie mit AP 1 behandelt; Konzentrationen in der Tankmischung: 0,1875 g/l Cyprodinil sowie $2,5 \times 10^6$ Zellen/ml DSM 14940 und $2,5 \times 10^6$ Zellen/ml DSM 14941; Ausbringung: 1.000 l/ha

Gruppe 8: mit Fludioxonil sowie mit AP 1 behandelt; Konzentrationen in der Tankmischung: 0,125 g/l Fludioxonil sowie $2,5 \times 10^6$ Zellen/ml DSM 14940 und $2,5 \times 10^6$ Zellen/ml DSM 14941; Ausbringung: 1.000 l/ha

Bei analoger Auswertung der Gruppen 1, 3 und 5 bis 8 wurde auch für die Anwendung der AP 1 Mischung gemeinsam mit nur einem der beiden chemischen Fungizide jeweils im Vergleich zur Einzelanwendung (chemische Fungizid bzw. AP 1 Mischung) eine synergistische Wirkung festgestellt, d.h. ein Synergismusfaktor von größer gleich 1,1 erzielt.

Die synergistische Wirkung von Mischungen von AP 1 mit den beiden chemischen Fungiziden Cyprodinil und Fludioxonil gegenüber Pathogenen wurde analog zu obigen Feldversuchen auch mit unterschiedlichen Konzentrationen an chemischem Fungizid bestätigt.

Für Cyprodinil konnte für allen Ausbringungsmengen, nämlich 20 g/ha; 100 g/ha; 187,5 g/ha; 375 g/ha, 500 g/ha und 1000 g/ha, die synergistische Wirkung bestätigt werden (Synergismusfaktor größer gleich 1,1), wobei für die Ausbringung von 187,5 g/ha und 375 g/ha jeweils der höchste Synergismusfaktor bestimmt wurde.

Für Fludioxonil konnte für alle Ausbringungsmengen, nämlich 10 g/ha; 50 g/ha; 125 g/ha; 250 g/ha; 500 g/ha und 1000 g/ha die synergistische Wirkung bestätigt werden (Synergismusfaktor größer gleich 1,1), wobei für die Ausbringung von 125 g/ha und 250 g/ha jeweils der höchste Synergismusfaktor bestimmt wurde.

**Patentansprüche**

1. Zubereitung enthaltend wenigstens ein chemisches Fungizid, **dadurch gekennzeichnet, dass** zusätzlich zu dem wenigstens einen chemischen Fungizid eine Mischung enthaltend wenigstens die Stämme *Aureobasidium pullulans* DSM 14940 und DSM 14941 enthalten ist, wobei das wenigstens eine chemische Fungizid sowie die Mischung der Stämme Aureobasidium pullulans Stämme in synergistisch wirkenden Mengen in der Zubereitung vorliegen und dass das wenigstens eine chemische Fungizid aus Fludioxonil oder einer Kombination enthaltend Fludioxonil und Cyprodinil gewählt ist.

2. Zubereitung nach Anspruch 1, **dadurch gekennzeichnet, dass** die *Aureobasidium pullulans* Stämme DSM 14940 und DSM 14941 in einem Zellmengenverhältnis von 2:1 bis 1:2-in der Zubereitung vorliegen.

3. Zubereitung nach Anspruch 1 oder 2, **dadurch gekennzeichnet, dass** als synergistisch wirkende Menge die *Aureobasidium pullulans* Stämme DSM 14940 und DSM 14941 jeweils in einer Konzentration von $1 \times 10^5$ bis $1 \times 10^8$ Zellen/ml Zubereitung vorliegen.

4. Zubereitung nach einem der Ansprüche 1, 2 oder 3, **dadurch gekennzeichnet, dass** als synergistisch wirkende Menge Cyprodinil in einer Konzentration von 0,00375 g/l Zubereitung bis 5 g/l Zubereitung enthalten ist.

5. Zubereitung nach einem der Ansprüche 1, 2 oder 3, **dadurch gekennzeichnet, dass** als synergistisch wirkende Menge Fludioxonil in einer Konzentration von 0,0025 g/l Zubereitung bis 5 gll Zubereitung enthalten ist.

6. Zubereitung nach einem der Ansprüche 1 bis 5, **dadurch gekennzeichnet, dass** Cyprodinil und Fludioxonil in einem Gewichtsverhältnis von 2:1 bis 1:2 in der Zubereitung vorliegen.

7. Zubereitung nach einem der Ansprüche 1 bis 6, **dadurch gekennzeichnet, dass** *Aureobasidium pullulans* DSM 14940 und DSM 14941 jeweils in einem Zellmengenverhältnis von $1 \times 10^6$ bis $2 \times 10^7$ Zellen/ml Zubereitung und die chemischen Fungizide Fludioxonil und Cyprodinil in einer Konzentration von 0,0025 g/l Zubereitung bis 1,0 g/l Zubereitung für Fludioxonil bzw. 0,00375 g/l Zubereitung bis 1,0 g/l Zubereitung für Cyprodinil enthalten sind.

8. Verwendung einer Zubereitung nach einem der Ansprüche 1 bis 7 zur Prophylaxe oder zur Reduktion der Ausbreitung von durch pilzliche Pathogene verursachten Pflanzenerkrankungen.

9. Verwendung nach Anspruch 8, **dadurch gekennzeichnet, dass** die pilzlichen Pathogene gewählt sind aus der Gruppe Neofabreaea spp. und Botrytis spp.

**EP 3 595 449 B1**

10. Verwendung nach Anspruch 8 oder 9, **dadurch gekennzeichnet, dass** die pilzlichen Pathogene gegenüber wenigstens einem chemischen Fungizid eine Resistenz und/oder verringerte Sensitivität aufweisen.

11. Verfahren zur Prophylaxe oder zur Reduktion der Ausbreitung von wenigstens einer durch einen pilzlichen Pathogen ausgelösten Pflanzenerkrankung, **dadurch gekennzeichnet, dass**

   a) wenigstens ein chemisches Fungizid gewählt ist aus Fludioxonil oder einer Kombination enthaltend Fludioxonil und Cyprodinil und
   b) eine Mischung enthaltend wenigstens die *Aureobasidium pullulans* Stämme DSM 14940 und DSM 14941

gemeinsam wenigstens einmal auf eine Kulturpflanze aufgebracht werden, wobei a) und b) in synergistisch wirksamen Mengen auf die Kulturpflanze aufgebracht werden.

12. Verfahren nach Anspruch 11, **dadurch gekennzeichnet, dass** a) und b) gemeinsam in einer Zubereitung gemischt, gelöst oder suspendiert werden und dass die Zubereitung auf die Kulturpflanze aufgebracht wird.

13. Verfahren nach Anspruch 11 oder 12, **dadurch gekennzeichnet, dass** die Zubereitung nach einem der Ansprüche 1 bis 7 eingesetzt wird.

14. Verfahren nach einem der Ansprüche 11, 12 oder 13, **dadurch gekennzeichnet, dass** pro Anwendung als synergistisch wirkende Menge jeweils $1 \times 10^{11}$ bis $1 \times 10^{14}$ Zellen/ha der *Aureobasidium pullulans* Stämme DSM 14940 und DSM 14941 aufgebracht werden.

15. Verfahren nach einem der Ansprüche 11 bis 14, **dadurch gekennzeichnet, dass** als das wenigstens eine chemische Fungizid Fludioxonil eingesetzt wird, wobei als synergistisch wirkende Menge 2,5 g/ha bis 5000 g/ha, aufgebracht werden.

16. Verfahren nach einem der Ansprüche 11 bis 15, **dadurch gekennzeichnet, dass** als zweites chemisches Fungizid Cyprodinil eingesetzt wird, wobei als synergistisch wirkende Menge 3,75 g/ha bis 5000 g/ha aufgebracht werden.

17. Verfahren nach einem der Ansprüche 11 bis 16, **dadurch gekennzeichnet, dass** die wenigstens eine Aufbringung des wenigstens einen chemischen Fungizids und der Mischung enthaltend wenigstens die *Aureobasidium pullulans* Stämme DSM 14940 und DSM 14941-während der Blütephase der Kulturpflanze erfolgt.

**Claims**

1. A preparation containing at least one chemical fungicide, **characterised in that** in addition to the at least one chemical fungicide, a mixture is contained which contains at least the strains of *Aureobasidium pullulans* DSM 14940 and DSM 14941, wherein the at least one chemical fungicide and the mixture of the strains *Aureobasidium pullulans* are present in the preparation in synergistically active quantities, and that the at least one chemical fungicide is selected from fludioxonil or a combination containing fludioxonil and cyprodinil.

2. The preparation according to claim 1, **characterised in that** the *Aureobasidium pullulans* strains DSM 14940 and DSM 14941 are present in the preparation in a cell quantity ratio of 2:1 to 1:2.

3. The preparation according to claim 1 or 2, **characterised in that**, as synergistically effective amount, the Aureobasidium pullulans strains DSM 14940 and DSM 14941 are each present in a concentration of $1 \times 10^5$ to $1 \times 10^8$ cells/ml preparation.

4. The preparation according to any one of claims 1, 2 or 3, **characterised in that**, as synergistically effective amount, cyprodinil is contained in a concentration of 0.00375 g/l preparation to 5 g/l preparation.

5. The preparation according to any one of claims 1, 2 or 3, **characterised in that**, as synergistically effective amount, fludioxonil is contained in a concentration of 0.0025 g/l preparation to 5 g/l preparation.

6. The preparation according to any one of claims 1 to 5, **characterised in that** cyprodinil and fludioxonil are present in the preparation in a weight ratio of 2:1 to 1:2.

19

7. The preparation according to any one of claims 1 to 6, **characterised in that** the Aureobasidium pullulans strains DSM 14940 and DSM 14941 are each present in a cell quantity ratio of $1\times106$ to $2\times107$ cells/ml preparation and the chemical fungicides fludioxonil and cyprodinil are contained in a concentration of 0.0025 g/l preparation to 1.05 g/l preparation for fludioxonil and 0.00375 gll preparation to 1.0 g/l preparation for cyprodinil.

8. Use of a preparation according to any one of claims 1 to 7 for prophylaxis and/or for reducing the spread of plant diseases caused by fungal pathogens.

9. The use according to claim 8, **characterised in that** the fungal pathogens are selected from the group of Neofabreaea spp. and Botrytis spp.

10. The use according to claim 8 or 9, **characterised in that** the fungal pathogens have a resistance and/or reduced sensitivity to at least one chemical fungicide.

11. A method for the prophylaxis or the reduction of the spread of at least one plant disease caused by a fungal pathogen, **characterised in that**

a) at least one chemical fungicide is selected from fludioxonil or a combination containing fludioxonil and cyprodinil and

b) a mixture containing at least the Aureobasidium pullulans strains DSM 14940 and DSM 14941

are applied jointly at least once to a crop plant, wherein a) and b) are applied in synergistically effective amounts to the crop plant.

12. The method according to claim 11, **characterised in that** a) and b) are mixed, dissolved or suspended jointly in a preparation, and **in that** the preparation is applied to the crop plant.

13. The method according to claim 11 or 12, **characterised in that** the preparation according to any one of claims 1 to 7 is used.

14. The method according to any one of claims 11, 12 or 13, **characterised in that**, as synergistically effective amount, in each case $1\times101$ 1 to $1\times1014$ cells/ha of the Aureobasidium pullulans strains DSM 14940 and DSM 14941 are applied.

15. The method according to any one of claims 11 to 14, **characterised in that** fludioxonil is used as the at least one chemical fungicide, wherein 2.5 g/ha to 5000 g/ha are applied as synergistically effective amount.

16. The method according to any one of claims 11 to 15, **characterised in that** cyprodinil is used as second chemical fungicide, wherein 3.75 g/ha to 5000 g/ha are applied as synergistically effective amount.

17. The method according to any one of claims 11 to 16, **characterised in that** the at least one application of the at least one chemical fungicide and of the mixture containing at least the Aureobasidium pullulans strains DSM 14940 and DSM 14941 is carried out during the flowering stage of the crop plant.

## Revendications

1. Préparation contenant au moins un fongicide chimique, **caractérisée en ce qu'**est contenu, en plus de l'au moins un fongicide chimique, un mélange contenant au moins les souches *Aureobasidium pullulans* DSM 14940 et DSM 14941, dans laquelle l'au moins un fongicide chimique ainsi que le mélange des souches d'Aureobasidium pullulans sont présents dans la préparation en des quantités agissant synergiquement, et que l'au moins un fongicide chimique est choisi parmi le fludioxonil ou une combinaison contenant du fludioxonil et du cyprodinil.

2. Préparation selon la revendication 1, **caractérisée en ce que** les souches *d'Aureobasidium pullulans* DSM 14940 et DSM 14941 sont présentes dans la préparation selon un rapport de quantité de cellules de 2:1 à 1:2.

3. Préparation selon la revendication 1 ou 2, **caractérisée en ce que** les souches *d'Aureobasidium pullulans* DSM 14940 et DSM 14941 sont respectivement présentes en tant que quantité agissant synergiquement en une con-

centration de $1 \times 10^5$ à $1 \times 10^8$ cellules/ml de préparation.

4. Préparation selon l'une des revendications 1, 2 ou 3, **caractérisée en ce que** le cyprodinil est contenu en tant que quantité agissant synergiquement en une concentration de 0,00375 g/l de préparation à 5 g/l de préparation.

5. Préparation selon l'une des revendications 1, 2 ou 3, **caractérisée en ce que** le fludioxonil est contenu en tant que quantité agissant synergiquement en une concentration de 0,0025 g/l de préparation à 5 g/l de préparation.

6. Préparation selon l'une des revendications 1 à 5, **caractérisée en ce que** du cyprodinil et du fludioxonil sont présents dans la préparation selon un rapport pondéral de 2:1 à 1:2.

7. Préparation selon l'une des revendications 1 à 6, **caractérisée en ce que** les souches *d'Aureobasidium pullulans* DSM 14940 et DSM 14941 sont respectivement contenues selon un rapport de quantité de cellules de $1 \times 10^6$ à $2 \times 10^7$ cellules/ml de préparation et les fongicides chimiques, le fludioxonil et le cyprodinil, sont contenus en une concentration de 0,0025 g/l de préparation à 1,0 g/l de préparation pour le fludioxonil ou de 0,00375 g/l de préparation à 1,0 g/l de préparation pour le cyprodinil.

8. Utilisation d'une préparation selon l'une des revendications 1 à 7 pour la prophylaxie ou la réduction de la propagation de maladies de plantes causées par des agents pathogènes fongiques.

9. Utilisation selon la revendication 8, **caractérisée en ce que** les agents pathogènes fongiques sont choisis parmi le groupe de Neofabreaea spp. et Botrytis spp.

10. Utilisation selon la revendication 8 ou 9, **caractérisée en ce que** les agents pathogènes fongiques présentent une résistance et/ou une sensibilité réduite par rapport à au moins un fongicide chimique.

11. Procédé de prophylaxie ou de réduction de la propagation d'au moins une maladie de plantes provoquée par un pathogène fongique, **caractérisé en ce que**

a) au moins un fongicide chimique est choisi parmi le fludioxonil ou une combinaison contenant du fludioxonil et du cyprodinil et
b) un mélange contenant au moins les souches *d'Aureobasidium pullulans* DSM 14940 et DSM 14941

conjointement est appliqué au moins une fois sur une plante cultivée, dans lequel la) et b) sont appliqués sur la plante cultivée en des quantités synergiquement actives.

12. Procédé selon la revendication 11, **caractérisé en ce que** a) et b) sont mélangés, dissous ou mis en suspension conjointement dans une préparation et que la préparation est appliquée sur la plante cultivée.

13. Procédé selon la revendication 11 ou 12, **caractérisé en ce que** la préparation selon l'une des revendications 1 à 7 est employée.

14. Procédé selon l'une des revendications 11, 12 ou 13, **caractérisé en ce que** par application, respectivement $1 \times 10^{11}$ à $1 \times 10^{14}$ cellules/ha des souches *d'Aureobasidium pullulans* DSM 14940 et DSM 14941 sont appliquées en tant que quantité agissant synergiquement.

15. Procédé selon l'une des revendications 11 à 14, **caractérisé en ce que** du fludioxonil est employé en tant que l'au moins fongicide chimique, dans lequel 2,5 g/ha à 5000 g/ha sont appliqués en tant que quantité agissant synergiquement.

16. Procédé selon l'une des revendications 11 à 15, **caractérisé en ce que** du cyprodinil est employé en tant que deuxième fongicide chimique, dans lequel 3,75 g/ha à 5000 g/ha sont appliqués en tant que quantité agissant synergiquement.

17. Procédé selon l'une des revendications 11 à 16, **caractérisé en ce que** l'au moins une application de l'au moins un fongicide chimique et du mélange contenant au moins les souches *d'Aureobasidium pullulans* DSM 14940 et DSM 14941 est effectuée pendant la période de floraison de la plante cultivée.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 69919762 T2
- EP 0930824 B1
- WO 9962341 A
- WO 2008114304 A

**In der Beschreibung aufgeführte Nicht-Patentliteratur**

- **R. CASTORIA et al.** *Postharvest Biology and Technology,* 2001, vol. 22, 7-17
- *CHEMICAL ABSTRACTS,* 121552-61-2
- *CHEMICAL ABSTRACTS,* 131341-86-1
- **COLBY et al.** *Weeds,* 1967, vol. 15, 20-22
- **LORENZ et al.** Phänologische Entwicklungsstadien der Weinrebe. *Vitic. Enol. Sci.,* 1994, vol. 49, 66-70
- **MEIER et al.** Phänologische Entwicklungsstadien des Kernobstes, des Steinobstes, der Johannisbeere und der Erdbeere. *Nachrichtenbl. Deut. Pflanzenschutz,* 1994, vol. 46, 141-153
- **S.R. COLBY.** Calculating synergistic and antagonistic responses of herbicide combinations. *Weeds,* 1967, vol. 15, 20-22
- **MEIER et al.** Phänologische Entwicklungsstadien des Kernobstes, des Steinobstes, der Johannisbeere und der Erdbeere. *Nachrichtenbl. Deut. Pflanzenschutz.,* 1994, vol. 46, 141-153